# EUROPEAN PATENT APPLICATION

(11) **EP 4 552 573 A1**
(43) Date of publication of application: **14.05.2025**
(21) Application number: 23835452.6
(22) Date of filing: 03.07.2023
(51) Int. Cl.: A61B 5/256

(54) **EARPIECE-TYPE ELECTRODE**

(30) Priority: 08.07.2022 JP 2022110766
(71) Applicant: NOK Corporation, Tokyo 105-8585 (JP)
(72) Inventor: KUROKO, Takanori, Fujisawa-shi, Kanagawa 251-0042 (JP); HAYASHI, Taisei, Fujisawa-shi, Kanagawa 251-0042 (JP); KUBO, Masayuki, Fujisawa-shi, Kanagawa 251-0042 (JP); HAYASHI, Takahiro, Fujisawa-shi, Kanagawa 251-0042 (JP)
(74) Representative: Global IP Europe Patentanwaltskanzlei
(86) International application number: PCT/JP2023/024537
(87) International publication number: WO 2024/009920

(57) **Abstract**

An earpiece-type electrode (1) to be inserted into an external auditory canal (2) includes a first truncated-cone wall portion (11) and a second truncated-cone wall portion (12) each formed of electrically conductive rubber and each having a substantially truncated-cone shape. The first truncated-cone wall portion (11) includes a first axial end portion (11a), and a second axial end portion (11b) larger in diameter than the first axial end portion (11a). The second truncated-cone wall portion (12) is larger in size than the first truncated-cone wall portion (11), and is arranged coaxially with the first truncated-cone wall portion (11). The second truncated-cone wall portion (12) includes a third axial end portion (12a) connected to the second axial end portion, and a fourth axial end portion (12b) larger in diameter than the third axial end portion (12a).

## Description

### FIELD

The present invention relates to an earpiece-type electrode. The earpiece-type electrode is inserted into an external auditory canal of a mammal including a human, or a bird.

### BACKGROUND

Japanese Patent Application Laid-Open Publication No. 2011-217986 discloses an electrode having a substantially cylindrical shape similar to an earpiece of a canal-type earphone. This electrode is inserted into an external auditory canal of a human to be used for supplying electric current to a skull or measuring a brain wave.

### BRIEF SUMMARY

### TECHNICAL PROBLEM

A earpiece of an earphone used for listening to music is formed of a soft material. Rubber hardness of the earpiece measured by a type-A durometer specified in JIS K 6253 (2012) is generally equal to or smaller than 30 degrees. Accordingly, the earpiece of the earphone can easily follow a shape and size of an external auditory canal which differ among individuals.

Meanwhile, an earpiece-type electrode includes a large amount of electrically conductive fillers mixed for enhancing an electrical conductivity of the electrode. For this reason, the earpiece-type electrode is formed of the hard material. Rubber hardness of the electrode measured by a type-A durometer is generally in a range from 60 degrees to 70 degrees. Thus, some persons feel discomfort such as oppression or pain in a case of wearing the earpiece-type electrodes for a long time.

It is preferable that the earpiece-type electrode stably contacts with an inner surface of an external auditory canal over a wide area at a certain degree of contact pressure, in order to secure electrical connection with the living body. Reducing hardness of the earpiece-type electrode causes the earpiece-type electrode to become more likely to buckle at the time of being put into the external auditory canal. Thus, there is a possibility of a decrease in a contact area between the earpiece-type electrode and the inner surface of the external auditory canal and a partial decrease in the contact pressure.

Since a shape and size of an external auditory canal differ among individuals, it is preferable to select an earpiece-type electrode having an appropriate size. An earpiece-type electrode having a size smaller than that of an external auditory canal can cause decreases in a contact area and a contact pressure between the electrode and an inner surface of the external auditory canal. An earpiece-type electrode having a size larger than that of an external auditory canal can cause a discomfort feeling. However, preparing earpiece-type electrodes of a large number of different sizes increases the cost.

An object of the present invention is to provide an earpiece-type electrode that appropriately fit into external auditory canals of various shapes and sizes.

### SOLUTION TO PROBLEM

A first aspect of the present disclosure provides an earpiece-type electrode to be inserted into an external auditory canal, including:
a first truncated-cone wall portion formed of electrically conductive rubber and having a substantially truncated-cone shape, the first truncated-cone wall portion including
   a first axial end portion and
   a second axial end portion larger in diameter than the first axial end portion; and
a second truncated-cone wall portion formed of electrically conductive rubber, larger in size than the first truncated-cone wall portion, having a substantially truncated-cone shape, and arranged coaxially with the first truncated-cone wall portion, the second truncated-cone wall portion including
   a third axial end portion connected to the second axial end portion, and
   a fourth axial end portion larger in diameter than the third axial end portion.

### ADVANTAGEOUS EFFECTS

An earpiece-type electrode according to the present invention can appropriately fit into external auditory canals of various shapes and sizes.

### BRIEF DESCRIPTION OF DRAWINGS

FIG. 1 is a sectional view illustrating a use example of an earpiece-type electrode according to an embodiment.
FIG. 2 is a front view of an earpiece-type electrode according to a first embodiment.
FIG. 3 is a perspective view of the earpiece-type electrode according to the first embodiment.
FIG. 4 is a sectional front view of the earpiece-type electrode according to the first embodiment.
FIG. 5 is a plan view of the earpiece-type electrode according to the first embodiment.
FIG. 6 is a front view of an earpiece-type electrode according to a second embodiment.
FIG. 7 is a perspective view of the earpiece-type electrode according to the second embodiment.
FIG. 8 is a sectional front view of the earpiece-type electrode according to the second embodiment.
FIG. 9 is a plan view of the earpiece-type electrode according to the second embodiment.

### DETAILED DESCRIPTION

The following describes various embodiments of the present invention with reference to the accompanying drawings. Reduction scales of the drawings are not necessarily precise, and some features are emphasized or omitted in the drawings in some cases.

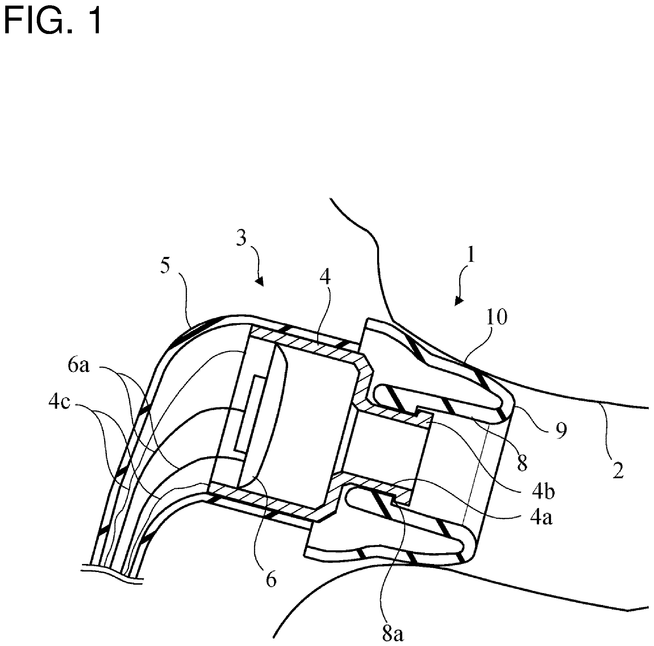

As illustrated in FIG. 1, an earpiece-type electrode 1 according to an embodiment is inserted into an external auditory canal 2 of a human, s different mammal, or a bird. The earpiece-type electrode 1 is formed of electrically conductive rubber. The earpiece-type electrode 1 is electrically connected to a measurement device (not illustrated) when used. The measurement device measures a brain wave or a blood flow.

In a use example illustrated in FIG. 1, the earpiece-type electrode 1 is mechanically and electrically connected to a connection component 3. The connection component 3 is mechanically and electrically connected to the measurement device. The connection component 3 includes a cylindrical component 4 made of metal. The cylindrical component 4 is fitted into a distal end of a protection tube 5 made of rubber.

A speaker 6 is fixed inside the cylindrical component 4. Wires 6a are provided for causing the speaker 6 to generate a sound. The wires 6a extend through an inside of the protection tube 5, and are electrically connected to the measurement device.

The cylindrical component 4 includes a smaller-diameter cylindrical portion 4a. A hook 4b is formed at an end portion of the smaller-diameter cylindrical portion 4a. The hook 4b is an annular flange that expands outward in the radial direction.

The earpiece-type electrode 1 includes an inner wall 8, a distal end wall 9, and an outer wall 10. The smaller-diameter cylindrical portion 4a is fitted into the inner wall 8. An annular hook 8a is formed at an end portion that is included in the inner wall 8 and that is located on an opposite side of the distal end wall 9. The hook 8a extends inward in the radial direction. The hook 8a is caught by the hook 4b. In this manner, the earpiece-type electrode 1 is fixed to the cylindrical component 4, and is electrically connected to the cylindrical component 4. The cylindrical component 4 is electrically connected to the measurement device via lead wires 4c extending through the inside of the protection tube 5. Releasing the mutually caught hooks 4b and 8a from each other enables the earpiece-type electrode 1 to be detached from the cylindrical component 4.

In this use example, the earpiece-type electrode 1 is used together with the speaker 6. In other words, the earpiece-type electrode 1 is used as an earpiece of a canal-type earphone. The speaker 6 generates a sound. The sound reaches a middle ear and an internal ear through an inner space of the smaller-diameter cylindrical portion 4a, an inner space of the inner wall 8, and the external auditory canal 2. The earpiece-type electrode 1 supplies electric current to the inner surface of the external auditory canal 2 with which the earpiece-type electrode 1 contact. The earpiece-type electrode 1 measures a change in an electrical property, such as a change in electric potential or electric current, received from the external auditory canal 2. The measurement device diagnoses a change in a brain wave or a blood flow, based on the change in the electrical property received from the external auditory canal 2. Thereby, a mental state of a subject who has heard a particular sound can be estimated.

In this use example, the speaker 6 is connected to the measurement device via the wires 6a. However, the speaker 6 may be wirelessly connected to the measurement device.

The speaker 6 may be omitted. The earpiece-type electrode 1 may be used for supplying electric current to a skull to electrically stimulate a vestibule, without generating a sound. Alternatively, the earpiece-type electrode 1 may be used for measuring a brain wave or a blood flow, without generating a sound.

The electrically conductive rubber that is a material of the earpiece-type electrode 1 includes one or both of particles and flakes. The particles and flakes are each an electrically conductive material. The rubber that is a matrix material is silicone rubber, for example. The electrically conductive material may be one of carbon black, gold (Au), platinum (Pt), silver (Ag), tungsten (W), molybdenum (Mo), copper (Cu), stainless steel, iron (Fe), and silver and silver chloride (Ag/AgCl), for example, or may be a mixture of materials selected from these.

### <First Embodiment>

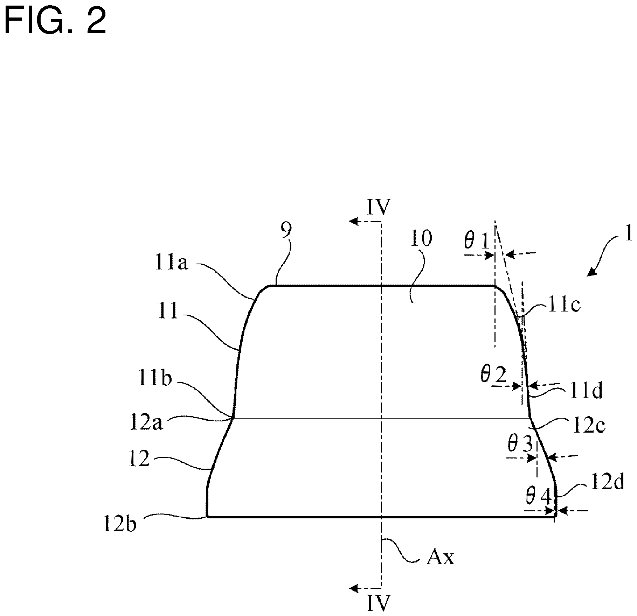

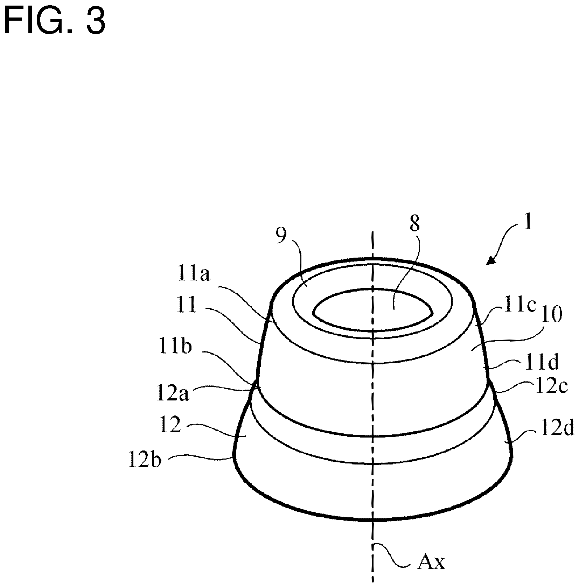

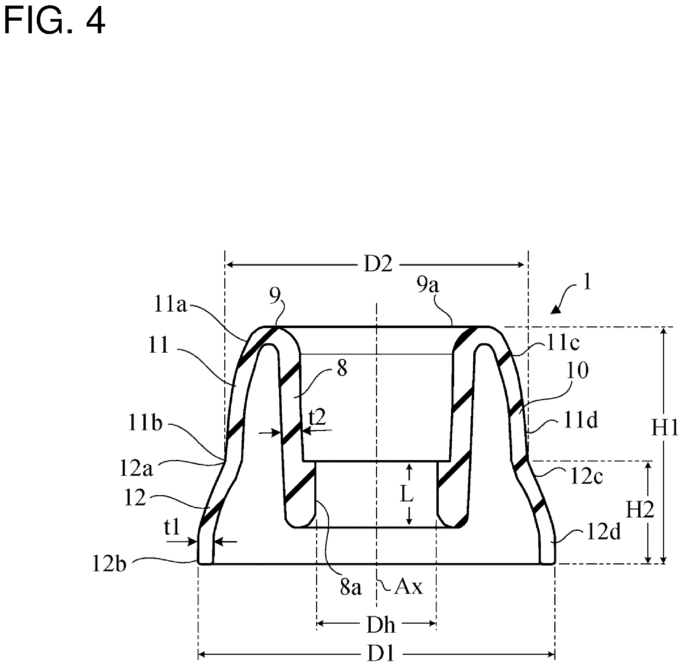

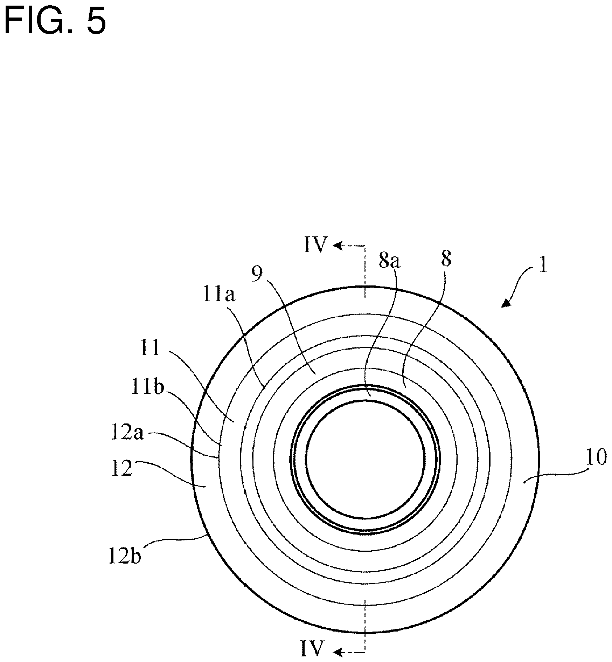

As illustrated in FIG. 2 to FIG. 5, the earpiece-type electrode 1 according to a first embodiment has a three-dimensional shape that is rotationally symmetric about the central axis Ax. FIG. 4 is a sectional view taken along the line IV-IV in FIG. 2 and FIG. 5. Since the earpiece-type electrode 1 has the rotationally symmetric shape, cutting the earpiece-type electrode 1 along any plane that includes the central axis Ax results in the same sectional view as that of FIG. 4.

The earpiece-type electrode 1 includes the inner wall 8, the distal end wall 9, and the outer wall 10. As illustrated in FIG. 1, when the earpiece-type electrode 1 is attached to a living body, the distal end wall 9 is oriented toward a deeper part of the external auditory canal 2.

The outer wall 10 contacts with the inner surface of the external auditory canal 2. The outer wall 10 includes a first truncated-cone wall portion 11 and a second truncated-cone wall portion 12. The first truncated-cone wall portion 11 and the second truncated-cone wall portion 12 each have a substantially truncated-cone shape. The first truncated-cone wall portion 11 and the second truncated-cone wall portion 12 may each have a completely truncated-cone shape. Alternatively, the first truncated-cone wall portion 11 and the second truncated-cone wall portion 12 may each have an incompletely truncated-cone shape, as in the wall portions 11 and 12 in FIG. 2 to FIG. 4. Thus, the first truncated-cone wall portion 11 and the second truncated-cone wall portion 12 may have a shape that is recognized as truncated cone at a glance and that is suitable for being inserted into the external auditory canal 2. In other words, the first truncated-cone wall portion 11 and the second truncated-cone wall portion 12 may each have even a shape of an umbrella or bowl whose radial-direction central portion is omitted. The truncated-cone wall portions 11 and 12 may each include one or both of a slightly concave portion and a slightly convex portion.

The first truncated-cone wall portion 11 includes a first axial end portion 11a and a second axial end portion 11b. The second axial end portion 11b has a diameter larger than that of the first axial end portion 11a. The first axial end portion 11a is smoothly connected to the distal end wall 9. The second axial end portion 11b is smoothly connected to the second truncated-cone wall portion 12.

As illustrated in FIG. 2, an inclination angle θ1 is larger than an inclination angle θ2. Here, the inclination angle θ1 is an inclination angle of a first part 11c with respect to the central axis Ax. The first part 11c is included in the first truncated-cone wall portion 11 and located on a side of the first axial end portion 11a. The inclination angle θ2 is an inclination angle of a second part 11d with respect to the central axis Ax. The second part 11d is included in the first truncated-cone wall portion 11 and located on a side of the second axial end portion 11b. The inclination angle θ1 of the first part 11c increases as a position approaches the first axial end portion 11a. Thus, the first truncated-cone wall portion 11 has an inclination angle that is an angle with respect to the central axis Ax and that increases as a position approaches the first axial end portion 11a. For this reason, the first truncated-cone wall portion 11 less irritates a subject when the earpiece-type electrode 1 is inserted into the external auditory canal 2.

The second truncated-cone wall portion 12 has a size larger than that of the first truncated-cone wall portion 11. The second truncated-cone wall portion 12 is arranged coaxially with the first truncated-cone wall portion 11. The second truncated-cone wall portion 12 includes a third axial end portion 12a and a fourth axial end portion 12b. The fourth axial end portion 12b has a diameter larger than that of the third axial end portion 12a. The third axial end portion 12a is smoothly connected to the second axial end portion 11b. The fourth axial end portion 12b is an end portion of the earpiece-type electrode 1, and is located on an opposite side of the distal end wall 9.

As illustrated in FIG. 2, an inclination angle θ3 is larger than an inclination angle θ4. Here, the inclination angle θ3 is an inclination angle of a third part 12c with respect to the central axis Ax. The third part 12c is included in the second truncated-cone wall portion 12 and located on a side of the third axial end portion 12a. The inclination angle θ4 is an inclination angle of a fourth part 12d with respect to the central axis Ax. The fourth part 12d is included in the second truncated-cone wall portion 12 and located on a side of the fourth axial end portion 12b. The fourth part 12d may have a cylindrical shape instead of a truncated-cone shape. In other words, the inclination angle θ4 may be zero. The inclination angle θ3 of the third part 12c is larger than the inclination angle θ2 of the second part 11d.

As illustrated in FIG. 4, the inner wall 8 has a substantially truncated-cone shape in which a cross-sectional area on a side of the distal end wall 9 is larger than a cross-sectional area on an opposite side. The inner wall 8 is arranged on an inner side of the outer wall 10 and coaxially with the outer wall 10. The annular hook 8a is formed at an end portion that is included in the inner wall 8 and that is on an opposite side of the distal end wall 9. The hook 8a extends inward in the radial direction.

The distal end wall 9 has a substantially arc shape in a section including the central axis Ax. The distal end wall 9 is smoothly connected to the inner wall 8 and the outer wall 10.

Dimensions of the earpiece-type electrode 1 are described with reference to FIG. 4. A height H1 of the earpiece-type electrode 1 is in a range from 7 mm to 11 mm, for example. Here, the height H1 is a distance along the axial direction between the fourth axial end portion 12b and a distal end surface 9a. A height H2 of the second truncated-cone wall portion 12 is in a range from 3 mm to 5 mm, for example. Here, the height H2 is a distance along the axial direction between the fourth axial end portion 12b and the third axial end portion 12a.

An outer diameter D1 of the earpiece-type electrode 1 is in a range from 10 mm to 14 mm, for example. Here, the outer diameter D1 is an outer diameter of the fourth axial end portion 12b. An outer diameter D2 of the first truncated-cone wall portion 11 is in a range from 9 mm to 12 mm, for example. Here, the outer diameter D2 is an outer diameter of the third axial end portion 12a, and is also an outer diameter of the second axial end portion 11b.

An inner diameter Dh of the hook 8a is in a range from 3 mm to 5 mm, for example. An axial length L of the hook 8a is in a range from 1 mm to 3 mm, for example.

A thickness t1 of the outer wall 10 is in a range from 0.3 mm to 0.6 mm, for example. A thickness t2 of the inner wall 8 is in a range from 0.5 mm to 0.8 mm, for example. The thickness t1 is preferably smaller than the thickness t2. In this case, the inner wall 8 has a higher rigidity, and thus prevents the earpiece-type electrode 1 from buckling. The distal end wall 9 and the outer wall 10 each have a lower rigidity, and thus deform in such a way as to follow a shape and size of the external auditory canal 2. For this reason, the distal end wall 9 and the outer wall 10 reduce irritation inflicted on a subject.

The earpiece-type electrode 1 according to the present embodiment includes the two truncated-cone wall portions 11 and 12 having mutually different sizes. Thereby, the earpiece-type electrode 1 can properly fit into the external auditory canals 2 having various shapes and sizes. Thus, the earpiece-type electrode 1 stably contacts with the inner surface of the external auditory canal 2 over a wide area at a certain degree of contact pressure. Necessity of preparing the earpiece-type electrodes 1 of a large number of different sizes can be reduced.

When the earpiece-type electrode 1 is attached to a living body, the first truncated-cone wall portion 11 is oriented toward a deeper part of the external auditory canal 2 in such a way as to be closer to the deeper part of the external auditory canal 2 than second truncated-cone wall portion 12. The first part 11c of the first truncated-cone wall portion 11 is oriented toward the deeper part of the external auditory canal 2 in such a way as to be closer to the deeper part of the external auditory canal 2 than the second part 11d. As illustrated in FIG. 2, the inclination angle θ1 of the first part 11c with respect to the central axis Ax is larger than the inclination angle θ2 of the second part 11d. Accordingly, the first truncated-cone wall portion 11 can be smoothly inserted into the external auditory canal 2. When a diameter of the external auditory canal 2 is small, the second part 11d is likely to come into surface contact with an inner surface of the external auditory canal 2 over a wide contact area.

When the earpiece-type electrode 1 is attached to a living body, the third part 12c of the second truncated-cone wall portion 12 is oriented toward a deeper part of the external auditory canal 2 in such a way as to be closer to the deeper part of the external auditory canal 2 than the fourth part 12d. The inclination angle θ3 of the third part 12c with respect to the central axis Ax is larger than the inclination angle θ2 of the second part 11d, and is larger than the inclination angle θ4 of the fourth part 12d. Thus, when a diameter of the external auditory canal 2 is large, the second truncated-cone wall portion 12 can be smoothly inserted into the external auditory canal 2. One or both of the third part 12c and the fourth part 12d are likely to come into surface contact with an inner surface of the external auditory canal 2 over a wide contact area.

### <Second Embodiment>

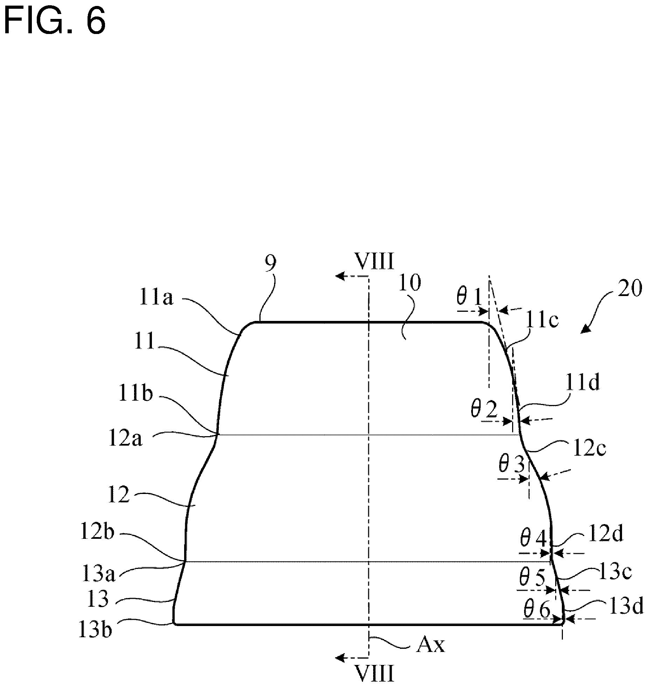

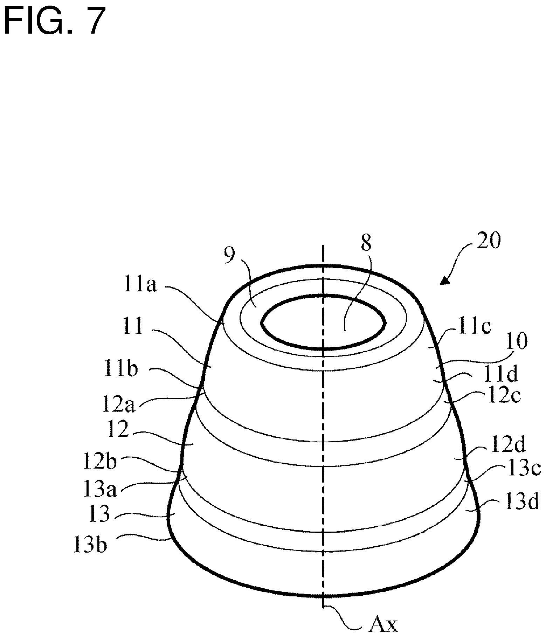

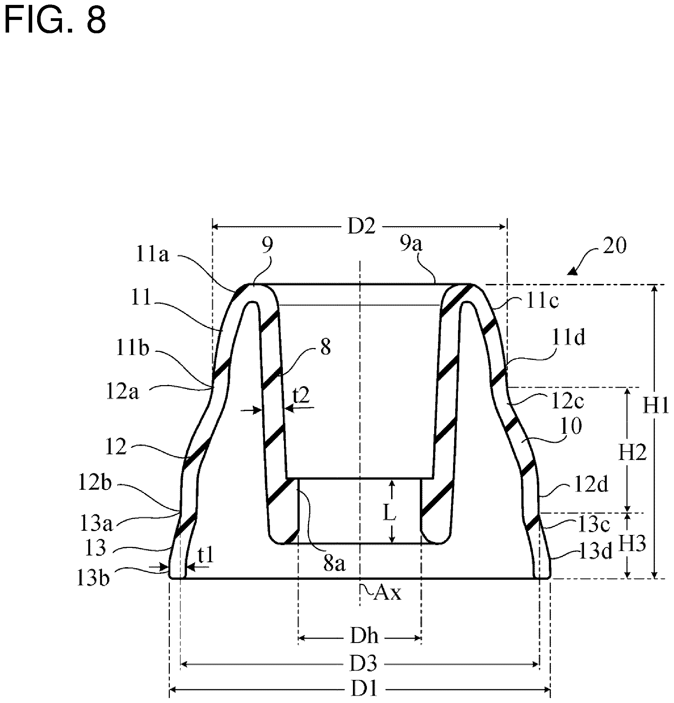

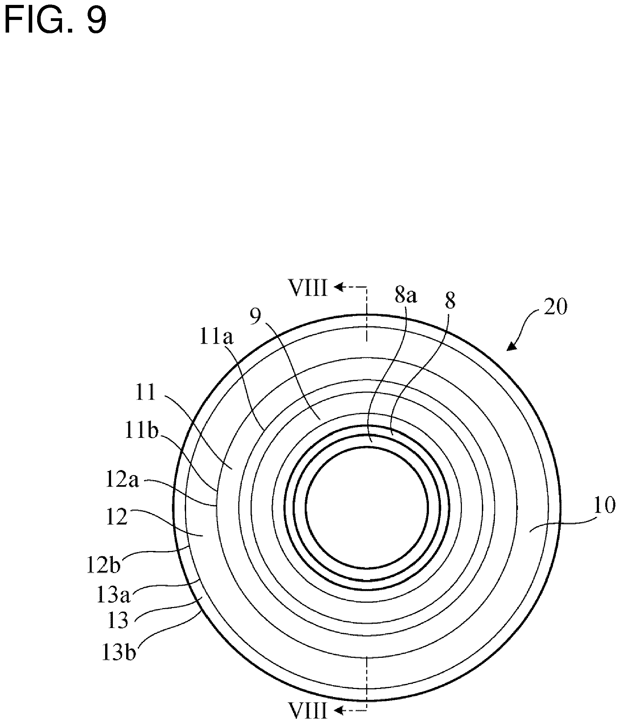

As illustrated in FIG. 6 to FIG. 9, an earpiece-type electrode 20 according to a second embodiment also has a three-dimensional shape that is rotationally symmetric about the central axis Ax. FIG. 8 is a sectional view taken along the line VIII-VIII in FIG. 6 and FIG. 9. Since the earpiece-type electrode 20 has the rotationally symmetric shape, cutting the earpiece-type electrode 20 along any plane that includes the central axis Ax results in the same sectional view as that of FIG. 8.

The earpiece-type electrode 20 includes features in common with the earpiece-type electrode 1 according to the first embodiment. Accordingly, description of these features is omitted.

The outer wall 10 in the present embodiment includes the first truncated-cone wall portion 11, the second truncated-cone wall portion 12, and a third truncated-cone wall portion 13. The third truncated-cone wall portion 13 has a size larger than that of the second truncated-cone wall portion 12. The third truncated-cone wall portion 13 has a substantially truncated-cone shape. The third truncated-cone wall portion 13 is arranged coaxially with the first truncated-cone wall portion 11 and the second truncated-cone wall portion 12. The third truncated-cone wall portion 13 includes a fifth axial end portion 13a and a sixth axial end portion 13b. The sixth axial end portion 13b has a diameter larger than that of the fifth axial end portion 13a. The fifth axial end portion 13a is smoothly connected to the fourth axial end portion 12b. The sixth axial end portion 13b is an end portion of the earpiece-type electrode 1, and is located on an opposite side of the distal end wall 9.

Dimensions of the earpiece-type electrode 20 are described with reference to FIG. 8. A height H1 of the earpiece-type electrode 20 is in a range from 9 mm to 13 mm, for example. Here, the height H1 is a distance along the axial direction between the sixth axial end portion 13b and the distal end surface 9a. A height H3 of the third truncated-cone wall portion 13 is in a range from 2 mm to 4 mm, for example. Here, the height H3 is a distance along the axial direction between the sixth axial end portion 13b and the fifth axial end portion 13a. The height H2 of the second truncated-cone wall portion 12 is in a range from 3 mm to 5 mm, for example. Here, the height H2 is a distance along the axial direction between the fourth axial end portion 12b and the third axial end portion 12a.

An outer diameter D1 of the earpiece-type electrode 20 is in a range from 11 mm to 15 mm, for example. Here, the outer diameter D1 is an outer diameter of the sixth axial end portion 13b. The outer diameter D2 of the first truncated-cone wall portion 11 is in a range from 9 mm to 12 mm, for example. Here, the outer diameter D2 is an outer diameter of the third axial end portion 12a, and is also an outer diameter of the second axial end portion 11b. An outer diameter D3 of the second truncated-cone wall portion 12 is in a range from 10 mm to 14 mm, for example. Here, the outer diameter D3 is an outer diameter of the fifth axial end portion 13a, and is also an outer diameter of the fourth axial end portion 12b.

An inner diameter Dh of the hook 8a is in a range from 3 mm to 5 mm, for example. An axial length L of the hook 8a is in a range 1 mm to 3 mm, for example.

A thickness t1 of the outer wall 10 is in a range from 0.3 mm to 0.6 mm, for example. A thickness t2 of the inner wall 8 is in a range from 0.5 mm to 0.8 mm, for example. The thickness t1 is preferably smaller than the thickness t2. In this case, the inner wall 8 has a higher rigidity, and thus prevents the earpiece-type electrode 20 from buckling. The distal end wall 9 and the outer wall 10 each have a lower rigidity, and thus deform in such a way as to follow a shape and size of the external auditory canal 2. For this reason, the distal end wall 9 and the outer wall 10 reduce irritation inflicted on a subject.

The earpiece-type electrode 20 according to the present embodiment includes the three truncated-cone walls 11, 12, and 13 having mutually different sizes. Thereby, the earpiece-type electrode 20 can properly fit into the external auditory canals 2 having various shapes and sizes. Thus, the earpiece-type electrode 20 stably contacts with the inner surface of the external auditory canal 2 over a wide area at a certain degree of contact pressure. Necessity of preparing the earpiece-type electrodes 20 of a large number of different sizes can be reduced.

When the earpiece-type electrode 20 is attached to a living body, the first truncated-cone wall portion 11 is oriented toward a deeper part of the external auditory canal 2 in such a way as to be closer to the deeper part of the external auditory canal 2 than second truncated-cone wall portion 12. The first part 11c of the first truncated-cone wall portion 11 is oriented toward the deeper part of the external auditory canal 2 in such a way as to be closer to the deeper part of the external auditory canal 2 than the second part 11d. As illustrated in FIG. 6, the inclination angle θ1 of the first part 11c with respect to the central axis Ax is larger than the inclination angle θ2 of the second part 11d. Accordingly, the first truncated-cone wall portion 11 can be smoothly inserted into the external auditory canal 2. When a diameter of the external auditory canal 2 is small, the second part 11d is likely to come into surface contact with an inner surface of the external auditory canal 2 over a wide contact area.

The inclination angle θ1 of the first part 11c increases as a position approaches the first axial end portion 11a. Thus, the first truncated-cone wall portion 11 has an inclination angle that is an angle with respect to the central axis Ax and that increases as a position approaches the first axial end portion 11a. For this reason, the first truncated-cone wall portion 11 less irritates a subject when the earpiece-type electrode 1 is inserted into the external auditory canal 2.

When the earpiece-type electrode 20 is attached to a living body, the third part 12c of the second truncated-cone wall portion 12 is oriented toward a deeper part of the external auditory canal 2 in such a way as to be closer to the deeper part of the external auditory canal 2 than the fourth part 12d. The inclination angle θ3 of the third part 12c with respect to the central axis Ax is larger than the inclination angle θ2 of the second part 11d, and is larger than the inclination angle θ4 of the fourth part 12d. Thus, when a diameter of the external auditory canal 2 is large, the second truncated-cone wall portion 12 can be smoothly inserted into the external auditory canal 2. One or both of the third part 12c and the fourth part 12d are likely to come into surface contact with an inner surface of the external auditory canal 2 over a wide contact area. The fourth part 12d may have a cylindrical shape instead of a truncated-cone shape. In other words, the inclination angle θ4 may be zero.

As illustrated in FIG. 6, an inclination angle θ5 is larger than an inclination angle θ6. Here, the inclination angle θ5 is an inclination angle of a fifth part 13c with respect to the central axis Ax. The fifth part 13c is included in the third truncated-cone wall portion 13 and located on a side of the fifth axial end portion 13a. The inclination angle θ6 is an inclination angle of a sixth part 13d with respect to the central axis Ax. The sixth part 13d is included in the third truncated-cone wall portion 13 and located on a side of the sixth axial end portion 13b. The inclination angle θ5 of the fifth part 13c is larger than the inclination angle θ4 of the fourth part 12d. Thus, when a diameter of the external auditory canal 2 is still larger, the third truncated-cone wall portion 13 can be smoothly inserted into the external auditory canal 2, and one or both of the fifth part 13c and the sixth part 13d are likely to come into surface contact with an inner surface of the external auditory canal 2 over a wide contact area. The sixth part 13d may have a cylindrical shape instead of a truncated-cone shape. In other words, the inclination angle θ6 may be zero.

Although the embodiments are described above, the present invention is not limited to these.

For example, the outer wall 10 may include four or more truncated-cone portions.

The inner wall 8, the distal end wall 9, and the outer wall 10 may be formed of the same material, or may be formed of different materials. For example, a matrix material used as a rubber material of each of the distal end wall 9 and the outer wall 10 that contact with a living body may be softer than a matrix material of the inner wall 8 that does not contact with a living body. In this case, the inner wall 8 has a higher rigidity, and thus prevents the earpiece-type electrode 1 from buckling. The distal end wall 9 and the outer wall 10 each have a lower rigidity, and thus deform in such a way as to follow a shape and size of the external auditory canal 2. For this reason, the distal end wall 9 and the outer wall 10 reduce irritation inflicted on a subject.

The earpiece-type electrodes 1 and 20 may be each used without the speaker 6 (FIG. 1). In this case, the distal end wall 9 does not need to include an opening through which a sound can propagate to an middle ear and an internal ear of a subject. The earpiece-type electrodes 1 and 20 may each include a solid core portion instead of the inner wall 8.

### Reference Signs List

- 1, 20: earpiece-type electrode
- 2: external auditory canal
- 8: inner wall
- 9: distal end wall
- 10: outer wall
- 11: first truncated-cone wall portion
- 11a: first axial end portion
- 11b: second axial end portion
- 11c: first part
- 11d: second part
- 12: second truncated-cone wall portion
- 12a: third axial end portion
- 12b: fourth axial end portion
- 12c: third part
- 12d: fourth part
- 13: third truncated-cone wall portion
- 13a: fifth axial end portion
- 13b: sixth axial end portion
- 13c: fifth part
- 13d: sixth part

## Claims

1. An earpiece-type electrode to be inserted into an external auditory canal, comprising:
a first truncated-cone wall portion formed of electrically conductive rubber and having a substantially truncated-cone shape, the first truncated-cone wall portion including
a first axial end portion and
a second axial end portion larger in diameter than the first axial end portion; and
a second truncated-cone wall portion formed of electrically conductive rubber, larger in size than the first truncated-cone wall portion, having a substantially truncated-cone shape, and arranged coaxially with the first truncated-cone wall portion, the second truncated-cone wall portion including
a third axial end portion connected to the second axial end portion, and
a fourth axial end portion larger in diameter than the third axial end portion.

2. The earpiece-type electrode according to claim 1, wherein
the first truncated-cone wall portion and the second truncated-cone wall portion each have a shape rotationally symmetric about a central axis.

3. The earpiece-type electrode according to claim 1 or 2, wherein
the first truncated-cone wall portion includes a first part located on a side of the first axial end portion and a second part located on a side of the second axial end portion, and an inclination angle of the first part with respect to a central axis is larger than an inclination angle of the second part with respect to the central axis, and
the second truncated-cone wall portion includes a third part located on a side of the third axial end portion and a fourth part located on a side of the fourth axial end portion, and an inclination angle of the third part with respect to the central axis is larger than the inclination angle of the second part, and is larger than an inclination angle of the fourth part with respect to the central axis.

4. The earpiece-type electrode according to claim 3, wherein
the inclination angle in the first part increases as a position approaches the first axial end portion.

5. The earpiece-type electrode according to any one of claims 1 to 4, further comprising:
a third truncated-cone wall portion formed of electrically conductive rubber, larger in size than the second truncated-cone wall portion, having a substantially truncated-cone shape, and arranged coaxially with the second truncated-cone wall portion, the third truncated-cone wall portion including
a fifth axial end portion connected to the fourth axial end portion, and
a sixth axial end portion larger in diameter than the fifth axial end portion.

6. The earpiece-type electrode according to claim 5, wherein
the first truncated-cone wall portion includes a first part located on a side of the first axial end portion and a second part located on a side of the second axial end portion, and an inclination angle of the first part with respect to a central axis is larger than an inclination angle of the second part with respect to the central axis,
the second truncated-cone wall portion includes a third part located on a side of the third axial end portion and a fourth part located on a side of the fourth axial end portion, and an inclination angle of the third part with respect to the central axis is larger than the inclination angle of the second part, and is larger than an inclination angle of the fourth part with respect to the central axis, and
the third truncated-cone wall portion includes a fifth part located on a side of the fifth axial end portion and a sixth part located on a side of the sixth axial end portion, and an inclination angle of the fifth part with respect to the central axis is larger than the inclination angle of the fourth part, and is larger than an inclination angle of the sixth part with respect to the central axis.
